(19) Europäisches Patentamt
European Patent Office
Office européen des brevets

(11) **EP 4 191 605 A1**

(12) **EUROPEAN PATENT APPLICATION**

(43) Date of publication:
**07.06.2023 Bulletin 2023/23**

(21) Application number: **22165163.1**

(22) Date of filing: **29.03.2022**

(51) International Patent Classification (IPC):
**G16H 40/40** (2018.01)    **G16H 40/67** (2018.01)

(52) Cooperative Patent Classification (CPC):
**G16H 40/40; G16H 40/67;** G16H 50/70

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.12.2021  US 202163285259 P**

(71) Applicant: **Koninklijke Philips N.V.**
**5656 AG Eindhoven (NL)**

(72) Inventors:
• **BASTIANELLI, Emanuele**
**Eindhoven (NL)**
• **GAO, Qi**
**Eindhoven (NL)**
• **CLOUT, Ramon, Antoine**
**Eindhoven (NL)**
• **BARBIERI, Mauro**
**Eindhoven (NL)**
• **AGTERDENBOS, Erik**
**Elndhoven (NL)**

(74) Representative: **Philips Intellectual Property & Standards**
**High Tech Campus 52**
**5656 AG Eindhoven (NL)**

(54) **CASE INTAKE SYSTEM AND METHOD WITH REMOTE DIAGNOSTIC TEST RECOMMENDATION AND AUTOMATIC GENERATION OF PROFILED QUESTIONS**

(57)    A non-transitory computer readable medium **(107, 111)** stores instructions executable by at least one electronic processor **(101, 113)** to perform a call intake method **(200)**. The method includes receiving caller-provided information describing an issue related to a functioning of a medical device from the caller; retrieving device log data automatically generated by the medical device; determining scores for diagnostic tests **(132)** of a set of diagnostic tests for diagnosing the issue with the medical device wherein the scores are determined based on the retrieved device log data and the caller-provided information; and outputting, on a display device **(105)** of a service engineer (SE) electronic device **(102)** operable by the SE, a ranked list **(142)** of one or more recommended diagnostic tests of the set of diagnostic tests based on the scores.

Fig. 2

EP 4 191 605 A1

**Description**

FIELD OF THE INVENTION

The following relates generally to the medical device maintenance arts, medical imaging device maintenance arts, medical device diagnostic arts, medical device diagnostic test recommendation arts, and related arts.

BACKGROUND OF THE INVENTION

A medical imaging system (such as magnetic resonance imaging (MRI), computed tomography (CT), interventional X-ray (iXR) and so forth) may occasionally malfunction, i.e., fail to work according to the specification. The problem is usually reported by hospital staff (e.g., technician, doctor, assistant) to the customer service centre. During the first customer call, the service engineer (SE) tries to get sufficient and accurate information about the reported problem and the status of the imaging device. This will help the SE to perform system diagnostics more effectively. However, the callers can spend only a limited amount of time reporting the problem. In addition, the ability of the callers to provide sufficient relevant information depends on their knowledge of the medical device. The SE, on the other hand, does have better knowledge about the imaging devices and has access to more data. The SE can request the latest log files, also known as Log-File-On-Demand (LFOD), from the imaging device. These log files may contain critical information for diagnosing the root cause of the problem. Some tests can be initiated by the SE to remotely to verify if a certain part of the system functions according to the specification. Both LFOD and remote tests can help the SE to ask the most relevant questions to the customer and identify the most probable malfunction areas.

While handling a case, the SE can build up her/his knowledge about the possible malfunction issue incrementally. The SE can run remote tests to this end, which provide evidence about functionalities/parts of the imaging device working correctly or not, helping the SE to exclude possible sources of malfunction. While running the tests, the SE also interacts with the customer on the phone, to acquire more information about the imaging device's behaviour. This often results in a sequence of questions, whose aim is to narrow down the possible causes for the malfunction. The problem here lies in the fact that the range of possible tests is quite wide, as the SE may have hundreds of available remote tests that can be run, and not all the tests are relevant (or likely to be relevant) to diagnosing the problem. The customer answers to SE's questions may exclude certain root causes and corresponding remote tests, and thus help in reducing the set of tests. Another non-negligible aspect of this process is that the customer's professional background varies considerably from one customer call to the next. For instance, a caller reporting a problem can be a local engineer, a technician (e.g., radiologist), or an administrator. SEs may not take this into account and ask questions that are too difficult for the caller, or fail to ask questions that an expert caller may be able to answer. The questions of the SE should match the caller's profile to be understood and therefore to be effective. Furthermore, time restriction bounds both the number of tests to run and the number of questions to ask.

The following discloses certain improvements to overcome these problems and others.

SUMMARY OF THE INVENTION

In one aspect, a non-transitory computer readable medium stores instructions executable by at least one electronic processor to perform a call intake method. The method includes receiving caller-provided information describing an issue related to a functioning of a medical device from the caller; retrieving device log data automatically generated by the medical device; determining scores for diagnostic tests of a set of diagnostic tests for diagnosing the issue with the medical device wherein the scores are determined based on the retrieved device log data and the caller-provided information; and outputting, on a display device of a service engineer (SE) electronic device operable by the SE, a ranked list of one or more recommended diagnostic tests of the set of diagnostic tests based on the scores.

In another aspect, a system includes a medical device, and at least one electronic processor programmed to receive caller-provided information describing an issue related to a functioning of a medical device from the caller; retrieve device log data automatically generated by the medical device; determine scores for diagnostic tests of a set of diagnostic tests for diagnosing the issue with the medical device wherein the scores are determined based on the retrieved device log data and the caller-provided information; and output, on a display device of a SE electronic device operable by the SE, a ranked list of one or more recommended diagnostic tests of the set of diagnostic tests based on the scores.

In another aspect, a call intake method includes receiving a request from a caller for assistance from a SE in resolving an issue with a medical device; receiving caller-provided information describing an issue related to a functioning of a medical device from the caller; retrieving device log data automatically generated by the medical device; determining scores for diagnostic tests of a set of diagnostic tests for diagnosing the issue with the medical device wherein the scores are determined based on the retrieved device log data and the caller-provided information; outputting, on a display device of a SE electronic device operable by the SE, a ranked list of one or more recommended diagnostic tests of the set of diagnostic tests based on the scores; and remotely controlling the medical device to perform the one or more performed diagnostic tests whereby the results are received from the medical device.

One advantage resides in minimizing an amount of time spent to resolve an issue with a medical device between a SE and a caller.

Another advantage resides in minimizing time to resolve a service issue for a medical device.

Another advantage resides in minimizing time to resolve a service issue for a medical device regardless of an experience level of a caller reporting the service issue.

Another advantage resides in minimizing time to resolve a service issue for a medical device by having a SE perform remote tests while on a service call with a caller.

A given embodiment may provide none, one, two, more, or all of the foregoing advantages, and/or may provide other advantages as will become apparent to one of ordinary skill in the art upon reading and understanding the present disclosure.

BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.

Fig. 1 diagrammatically illustrates an illustrative customer call intake system for collecting information and recommending remote test for supporting servicing of a medical device in accordance with the present disclosure.
Figs. 2 and 3 show exemplary flow chart operations of the system of Fig. 1.
Figs. 4 and 5 show examples of a user interface of the system of Fig. 1.

DETAILED DESCRIPTION OF EMBODIMENTS

The following relates to improved customer service call intake. Presently, a service engineer (SE) takes a call (phone, videocall, texting conversation) from a customer reporting a problem with a medical device. The employee or other person representing the customer who places the call to the SE is referred to herein as the caller. The SE asks the caller questions and runs remote diagnostic tests on the medical device at issue, with the goal of remotely resolving the problem or at least collecting information to assist a field service engineer who may be dispatched to the site. The SE may use a limited script of standard questions, for example including questions about patient involvement required by applicable regulations, and possibly a written diagnostic workflow, and his or her own expertise, in selecting the questions to ask the caller and remote tests to run on the medical device. However, as there may be hundreds of available remote tests that could be run, this approach is inefficient.

In some embodiments disclosed herein, a diagnostic test recommender receives information including any previous diagnostic test results, log file on demand (LFOD) data, and caller answers to questions, and outputs a ranked list of diagnostic tests.

In a variant embodiment, a question recommender operates on similar information to provide a ranked list of questions to ask the caller. The question recommender is implemented using a database of question templates (analogous to the database of available diagnostic tests that are ranked by the test recommender). Additionally, since the caller with which the SE is communicating may vary in expertise from an office assistant to a technician to an engineer, this level of expertise is determined early on (e.g., as an initial question to the caller) and serves as a further input to the question recommender. The level of expertise is used as an initial filter, e.g., if the expertise is office assistant, then many of the questions whose answers require higher level of expertise can be quickly filtered out.

In one embodiment, the test (and optional question) recommender can be implemented separately from the remote medical system interface that is used for connecting with the medical device to actually perform remote tests. Alternatively, they can be integrated together.

In other embodiments, the test recommender may be provided to the caller (in instances in which the caller has sufficient technical qualifications) so that the caller can run recommended remote diagnostic tests directly. In various levels of such caller cooperation, the caller may in one variant passively see a mirror of the test recommender; in another the caller may share the test recommender with the SE so that either caller or SE can initiate a remote diagnostic test; while in yet another the caller alone sees the test recommender. In this latter case a remote assistant is implemented for use by the caller in self-diagnosing a problem. These are also not necessarily mutually exclusive options.

In another aspect, the recommender system can also prefilter the LFOD data, so that only portions of the LFOD data that may be relevant to the problem are transferred to the SE. This can save bandwidth.

While illustrative embodiments are described in the following primarily in terms of medical imaging devices, the disclosed systems and methods are broadly applicable to any medical device which automatically maintains log files and can perform remote diagnostic tests. For example, these include medical imaging devices (including image guided therapy, (IGT)), patient monitors, mechanical ventilators and other respiratory assistance devices, radiation therapy (RT) devices, and even more broadly could encompass software-based medical devices such as a complex RT planning system.

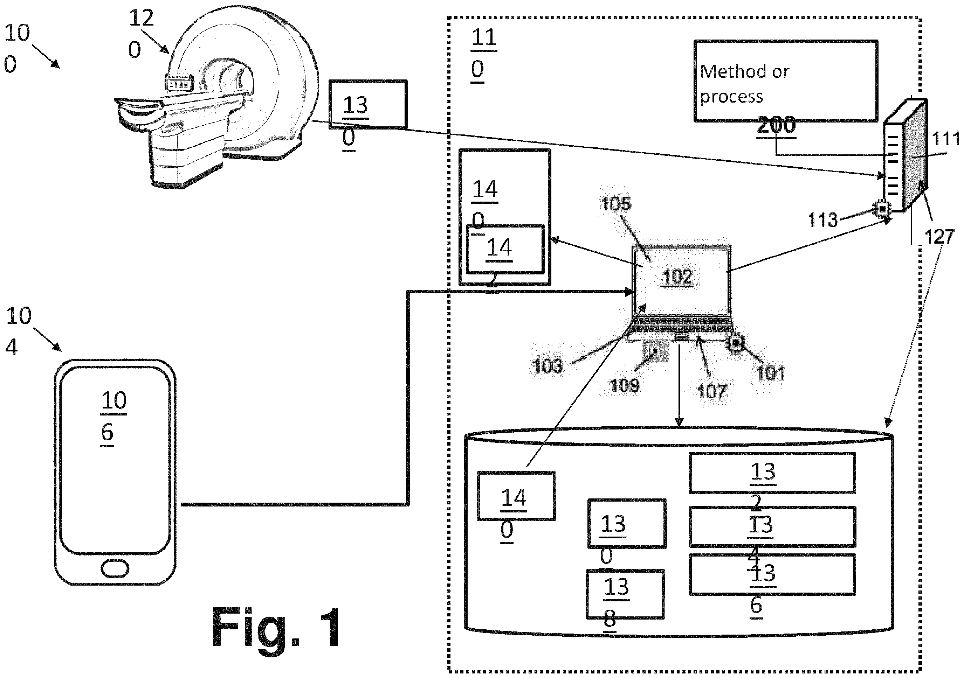

With reference to Fig. 1, an illustrative call intake system 110 for supporting a service engineer (SE) in servicing a device 120 (e.g., a medical imaging device - also referred to as a medical device, an imaging device, imaging scanner, and variants thereof) is diagrammatically shown. By way of some non-limiting illustrative examples, the medical imaging device under service may be a magnetic resonance imaging (MRI) scanner, a computed tomography (CT) scanner, a positron emission tomography (PET) scanner, a gamma camera for performing single photon emission computed tomography (SPECT), an interventional radiology (IR) device, or so forth. (More generally, the disclosed approach can be applied in conjunction with any type of computerized device that automatically generates log data that are analyzed by predictive models to predict component failures, e.g., the approach could be applied to a commercial airliner, radiation therapy device, or so forth). Although shown in FIGURE 1 as an imaging device, the medical device 120 can also be any other suitable medical device, such as an image guided therapy (iGT) device, a radiation therapy device, and so forth.

As shown in Fig. 1, a caller placing a call to report an issue (i.e., problem) with the medical device 120 uses a caller electronic device 104 that may, for example, be a cellphone (as shown in FIGURE 1) or landline telephone or VOIP phone in the case of a telephonic call, or a workstation or electronic device or mobile device (cellphone, tablet computer, et cetera) in the case of a call placed by telephone, text messaging or video conferencing. The caller electronic device 104 is used by a caller who represents the customer, that is, the hospital or other entity that owns the medical device 120. The caller may, by way of nonlimiting illustrative example, be an employee of the customer, or an independent contractor working for the customer, or so forth. Depending on the customer's practices, available staffing, and the specific situation leading to the call being placed, the caller could have a wide range of expertise in the medical device 120 that is the subject of the call, ranging from being a secretary, receptionist, or other office worker with little or no knowledge of the medical device 120 to a medical professional or engineer with extensive knowledge of the medical device 120 and its operation. In some embodiments, the caller electronic device 104 may for example be a portable device such as a notebook computer that is carried or accessed by the caller. In other embodiments, the caller electronic device 104 may be an imaging system controller or computer integral with or operatively connected with the medical device 120 undergoing service (e.g., at a medical facility). In another example, the caller electronic device 104 may be a computer based at the hospital.

The caller electronic device 104 includes a display 106 via which text or images can be displayed, and includes a communication interface such as, for example, a cellular telephone network (e.g. a 3G, 4G, or 5G cellular network), a voice over internet protocol (VOIP) connection, or so forth, and may provide for the call from the caller to the SE to be placed by way of a telephone connection, by text messaging, by a video call, or so forth.

Fig. 1 also shows an SE electronic device 102 operable by the SE at a vendor facility and included with or in communication with the call intake system 110. While a single SE electronic device 102 is shown, in a typical service center there may be a staff of SE's each using his or her own SE electronic device, of which the illustrative SE electronic device 102 is one illustrative example. The SE electronic device 102 may for example be a workstation used by an SE, such as a remote SE (RSE) or a field SE (FSE). In another example, the SE electronic device 102 may be a portable device such as a notebook computer that is carried or accessed by an RSE. The SE electronic device 102 can be a desktop computer or a personal device, such as a mobile computer system such as a laptop or smart device. In other embodiments, the SE electronic device 102 may be an imaging system controller or computer integral with or operatively connected with the imaging device undergoing service (e.g., at a medical facility). As another example, the SE electronic device 102 may be a portable computer (e.g., notebook computer, tablet computer, or so forth). In another example, the SE electronic device 102 may be the controller computer of the imaging device under service, or a computer based at the hospital. In other embodiments, the service device may be a mobile device such as a cellular telephone (cellphone) or tablet computer.

The SE electronic device 102 includes a display 105 via which alerts generated by predictive failure models are displayed, along with likely root cause and service action recommendation information as disclosed herein. The SE electronic device 102 also preferably allows the service engineer to interact with the servicing support system via at least one user input device 103 such a mouse, keyboard, or touchscreen. The service device further includes an electronic processer 101 and non-transitory storage medium 107 (internal components which are diagrammatically indicated in Fig. 1). The non-transitory storage medium 107 stores instructions which are readable and executable by the electronic processor 101 for interfacing with the servicing support system 100. The SE electronic device 102 also includes a communication interface 109 to communicate with a backend server or processing device 111, which typically implements the computational aspects of the servicing support system 100 (e.g., the server 111 has the processing power for implementing computationally complex aspects of the servicing support system 100). Such communication interfaces 109 include, for example, a wired and/or wireless Ethernet interface (e.g., in the case in which the SE electronic device 102 is a RSE workstation). Some aspects of the call intake system 110 may also be implemented by cloud processing or other remote processing (that is, the server computer 111 may be embodied as a cloud-based computing resource comprising a plurality of interconnected servers).

Illustrative Fig. 1 further shows the call intake system 110 (e.g., implemented and/or owned by the imaging device vendor or leased by the vendor from a cloud computing service provider). The call intake system 110

receives log data (e.g., a machine log automatically generated by the medical imaging device **120**, a service log for the medical imaging device **120**, and/or so forth) on a continuous or occasional basis (e.g., in some setups the imaging device **120** uploads machine log entries to the call intake system **110** on a daily basis). The call intake system **110** further includes a backend server **111** equipped with an electronic processor **113** (diagrammatically indicated internal component). The server **111** is equipped with non-transitory storage medium **127** (internal components which are diagrammatically indicated in Fig. 1). While a single server computer is shown, it will be appreciated that the call intake system **110** may more generally be implemented on a single server computer, or a server cluster, or a cloud computing resource comprising ad hoc-interconnected server computers, or so forth. Furthermore, while Fig. 1 shows a single medical imaging device **120,** more generally the call intake system **110** will handle customer calls for many customers (e.g., tens, hundreds, or more hospitals, radiology centers, and so forth) and for many medical imaging devices (e.g., tens, hundreds, or more imaging devices) and performs the disclosed processing for each such customer call to the call intake system **110**. In the case in which the call intake system **110** comprises a server computer **111** with substantial processing capability, the server computer **111** may optionally provide other functionality in support of the medical device servicing center or department, such as running predictive diagnostic models on incoming log entries to provide early detection of incipient problems, providing various types of business support (scheduling, record-keeping, et cetera), or so forth, in addition to providing processing for the call intake system **110**.

With continuing reference to Fig. 1, the non-transitory computer readable medium **127** stores log files **130** received from the medical device **120**. The non-transitory computer readable medium **127** also stores a set of diagnostic tests **132** executable by the SE via the service device **102** for diagnosing an issue with the medical device **120**. Moreover, the non-transitory computer readable medium **127** stores a set of question templates **134** for use with received questions about an issue with the medical device **120**. In some examples, the non-transitory computer readable medium **127** stores a set of questions **136** related to issues with the medical device **120**.

The non-transitory storage medium **127** stores instructions executable by the electronic processor **113** of the backend server **111** to perform a call intake method **200** for assisting the SE in handling incoming customer calls reporting problems with medical devices, including addressing an issue with the illustrative medical device **120**. In general, the SE receives a customer call via the SE electronic device **102** from a caller representing the customer using the caller electronic device **104**. The goal of the SE in taking the call is to efficiently collect information from the caller, and to concurrently select and run a subset of a set of diagnostic tests **132** that can be remotely run by the SE on the medical imaging device **120**. The number of available diagnostic tests in the set of diagnostic tests **132** for a complex medical device such as an MRI scanner, CT scanner, radiation therapy device, or so forth may number in the hundreds, with only some of these diagnostic tests being likely to be useful in diagnosing the current problem with the medical device **120**. Similarly, only some questions that could be posed to the caller are likely to collect useful information, and depending on the expertise of the caller only some of those questions are likely to be able to be answered by the caller if the caller has limited (or no) expertise on the medical device **120**. In view of these difficulties, the call intake method **200** implemented using the call intake system **110** is designed to recommend optimal diagnostic tests to run, and in some embodiments to also recommend optimal questions to pose to the caller.

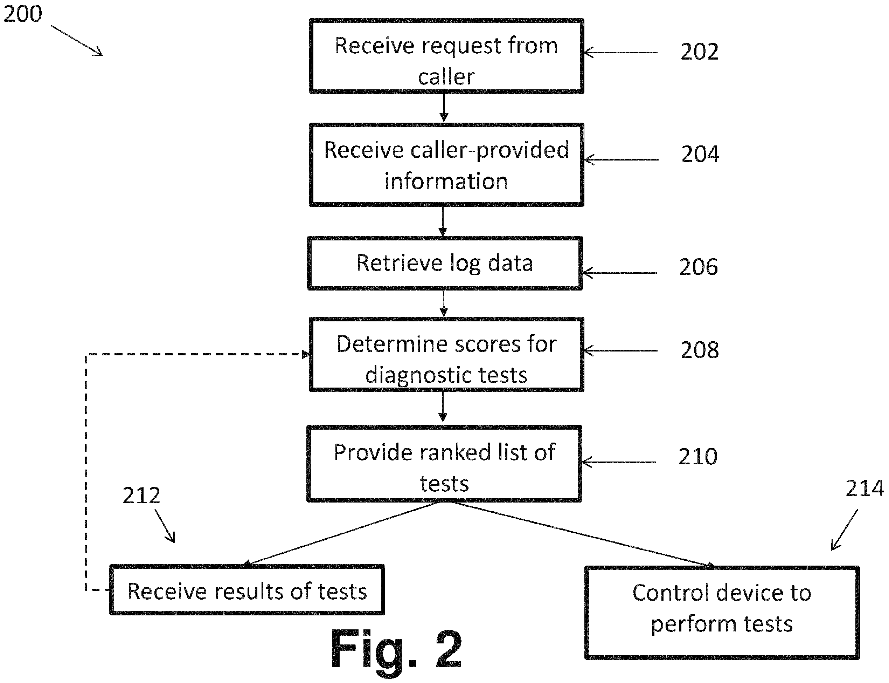

With continuing reference to Fig. 1 and further reference to Fig. 2, an illustrative embodiment of the method **200** executable by the electronic processor **113** is diagrammatically shown as a flowchart. In some examples, the method **200** may be performed at least in part by cloud processing.

To begin the method **200,** at an operation **202,** a request from a caller of the medical device **120** for assistance from an SE in resolving an issue with the medical device **120** can be received at the SE electronic device **102**. In response, at an operation **204,** the SE receives the caller-provided information that describes an issue related to a functioning of the medical device **120,** and opens a docket, file, case, or other electronic data structure on the backend server **111** to record the new matter. This may entail, for example, bringing up a call intake form presented on the SE electronic device **102** with fields for various relevant pieces of information, e.g., intake date/time, customer information, make/model/serial number of the medical device **120,** problem description, and so forth. Some of this information may be entered into structured fields, e.g., the model and serial number may be structured fields formatted in accordance with the vendor's standardized model and serial number format. Other information may be freeform text entry fields, e.g., the problem description. Some information may be retrieved from the backend server **111**. For example, upon entry of a customer identifier other customer information such as location may be retrieved from a customer database and auto-populated into the intake form. At an operation **206,** after the SE has entered information sufficient to identify the specific medical device **120** having the problem, the device log data **130** automatically generated by the medical device **120** is retrieved from the backend server **111**.

In some embodiments, based on the information being entered into the call intake system **110** by the SE and further based on results of diagnostic tests (once such tests begin to be run), the call intake system may display a list of questions selected from the set of questions **136** on the SE electronic device **102**. The SE can then decide whether to pose these questions to the caller.

In some examples, an indication of a level of expertise of the caller can be received from the caller in response to a question posed by the SE, and questions from the list of questions **136** can be filtered out based on the level of expertise of the customer.

At an operation **208**, scores **138** for diagnostic tests of the set of diagnostic tests **132** are determined for diagnosing the issue with the medical device. The scores **138** are determined based on the retrieved log data **132**, and the customer provided information (i.e., the answers to the questions of the set of questions **136** which are posed to the caller by the SE).

At an operation **210**, a user interface (UI) **140** is generated at the SE electronic device **102** for display on the display device **105**. The UI **140** shows a ranked list **142** of one or more recommended diagnostic tests of the set of diagnostic tests **132** based on the scores **138**.

The SE then implements the recommended diagnostic tests (or some subset thereof chosen by the SE). The implementation may entail the SE operating a separate remote interface with the medical device **120** to remotely perform the tests. In some embodiments, the remote interface may be integrated with the call intake system **110**, in which case the SE may perform the tests directly via the user interface of the call intake system **110**. Some diagnostic tests may be performed entirely automatically and remotely by the SE. Other diagnostic tests may be remotely performed by the SE, but with on-site assistance from a radiology technician or other on-site operator of the medical device **120**. For example, if a diagnostic test entails imaging a specific imaging phantom, then the SE may coordinate with the on-site operator to have the on-site operator load the imaging phantom into the examination region of the imaging device **120**. In some embodiments, at an operation **212**, results for one or more performed diagnostic tests of the one or more recommended diagnostic tests **132** are received by the backend server **111**. If a separate remote interface is used to perform the tests, then the results may come back to that separate remote interface and then the operation **112** involves manual copying of the results by the SE from the separate remote interface into the call intake system **110**. On the other hand, if the remote interface is integrated with the call intake system **110**, then in the operation **212** the test results may be directly received by the call intake system **110**. The performed diagnostic tests are then removed from the set of diagnostic tests **132** to generate a set of remaining diagnostic tests. The scores **138** for the diagnostic tests of the set of remaining diagnostic tests are updated based the retrieved device log data, the customer-provided information, and the received results for the performed diagnostic tests. An updated ranked list **142** of remaining recommended diagnostic tests is then provided on the UI **140** of the customer service device **102** based on the updated scores.

In other embodiments, at an optional operation **214**, the medical device **120** can be remotely controlled by the SE electronic device **102** to perform the one or more performed diagnostic tests, in which the results are received from the medical device **120**.

In another embodiment, the updated ranked list **142** can provide the remaining recommended diagnostic tests, along with a required experience level to perform these tests. These tests can then be queued based on the required experience level for each test. The updated ranked list **142** (with the required experience level for each test) can then be tailored and sent to each SE based on the experience of the SE who can perform the required tests. For example, a first test "A" and a second test "B" can be performed by the caller. The caller can then input information ("C"), and perform a third test "D", for which assistance from the SE is required. The SE can input a response "E" to resolve the issue for the third test "D". If the response "E" does not resolve the issue for the third test "D", then the SE can input a second response "G" to try and resolve the issue for the third test "D". The SE can then download new log data **130** from the medical device **120** based on these tests and responses, and provide next steps for the caller to perform.

EXAMPLES

The following describes examples of the servicing support system **300**. A test recommender module **302** is implemented into the backend server **111**, and is a standalone system capable of suggesting remote diagnostic tests to run on the inspected medical device **120** to the SE, based on the information extracted from the retrieved log data **130**, also referred to as log file on demand (LFOD).

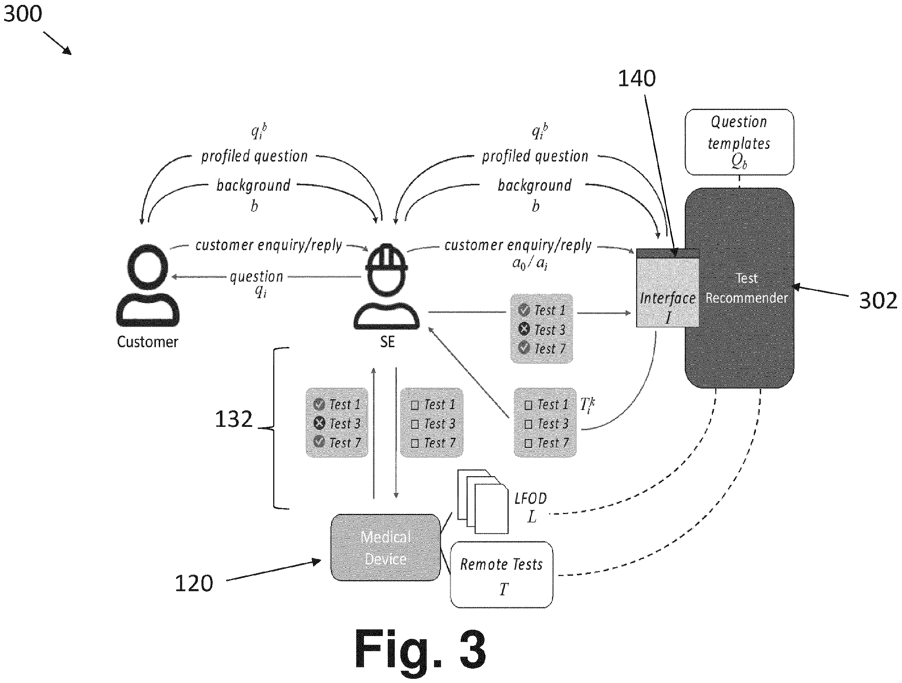

Fig. 3 shows an example of the the servicing support system **300**. The whole process is iterative, where the SE starts by providing the user enquiry to the test recommender module **302**. It uses this input plus the information in the LFOD **130** to select a subset of plausible remote tests to run. The SE then launches one or more of these tests, selecting them also on the base of her/his experience. The results of the tests are then presented to the test recommender module **302**. In parallel, the SE keeps asking questions to the customer, whose answers are provided to the test recommender module **302** together with the test results, to improve the test selection process. The whole process is iteratively repeated until a malfunction area is identified. In addition, the test recommender module **302** can also suggest further discriminant questions for the customer to the SE. These are selected from a set of customer and profile-specific templates based on the test results and previous interaction(s). The SE must provide the customer profile as input to the test recommender module **302** at the beginning of the process.

The interaction between the customer and the SE can be formalised as the sequence $a_0 ..., a_n$ of customer answers to SE questions, represented by textual inputs. Alternatively, SE questions can be considered in the formalization, i.e., the element of the sequence at the $i$th iteration of the interaction is a pair $(q_i, a_i)$ with $i =1...,n$. The answer $a_0$ corresponds to the customer initial enquiry.

The interaction starts with the customer enquiry, which describes an occurring malfunction. Through an interface $I$ (i.e., the UI **140**), the SE then inserts the user enquiry $a_0$, suitably summarised and processed, into the Test Recommender. After analysing both the user enquiry and the LFOD **130**, the system outputs a set of remote tests $T^k$ to run on an output dial of the same interface I. The SE runs the tests and/or in parallel asks further questions to the customer. After receiving the test results, the SE reiterates the process, providing the test recommender module **302** also with the test results again through the interface I. Additional user responses $a_i$ can be provided at every iteration $i$.

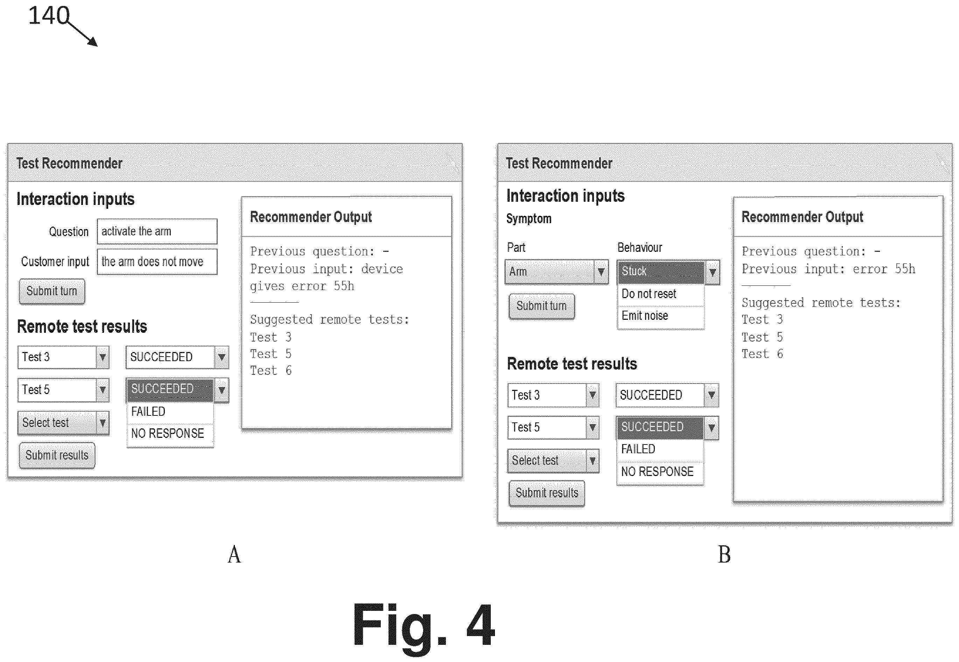

The interface $I$ presents input mechanisms to accept the textual representations of the answer $a_i$ (and additionally the corresponding question $q_i$), as well as to prompt the results of remote tests. Such mechanisms can be implemented in several ways. Textual inputs can be inserted as plain text, or with additional multiple-choice menus when enough structured knowledge about possible device malfunctions is available. Test results can be inserted through pre-defined multiple-choice menus, where the finite set of known test outcomes can be associated to each test in the selected subset of tests $T^k$. The test recommender module **302** may be implemented to directly communicate with the diagnosed medical device **120,** so that it would be possible to run remote tests and get the output directly in the test recommender module **302** interface. In such a way, the SE may just select the tests to run from the interface I, and the results may be automatically notified to the test recommender module **302,** without the SE having to insert them manually. Fig. 4 shows examples of the interface I (i.e., the UI **140).**

The functioning of the test recommender module **302** can be formalised as a scoring function according to Equation 1:

$$f(a_i, L, R_i^k) = s_1..., s_m, \qquad (1)$$

where $s_1..., s_m$ are the scores associated with each test $t \in T,$ with $m = |T|$, where each $s$ can attain values from a discrete or continuous set, and $R_i^k$ is the results of the $k$ tests $T_i^k$ run at iteration $i$. Results can be either binary, i.e., SUCCEEDED/FAILED, or n-ary/continuous, e.g., the specific error code or output message.

The scoring function $f$ can either jointly provide scores over the whole set $T,$ e.g., a likelihood score of each test given the inputs, or independently score each test $t \in T$ against the arguments. In this latter case, it would be formalised as Equation 2:

$$f(a_i, L, R_i^k, t_l) = s_l, \qquad (2)$$

where $t_l$ is the $l$th test in $T$. In either case, each test $t \in T$ is paired with a score s, providing a final rank of tests. The subset of possible tests $T^k$ can be built by either selecting the top k tests, or by applying a threshold on the scores or a combination of both.

The choice of the tests in $T_i^k$ does not depend only on the instant information available at iteration $i$, but also on the historical context of the interaction. This comprises two parts: the outcomes of the tests run so far, and the history of the customer-SE verbal interaction. To include the historical context, the scoring function for the i-th iteration can be extended to Equation 3:

$$f(a_i, L, R_i^k, A_i, E_i) = s_1,..., s_m, \qquad (3)$$

where $A_i = a_0..., a_{i-1}$ (or alternatively $a_0, (q_i, a_1) ..., (q_{i-1}, a_{i-1})$ if SE questions are also considered when modelling the interaction), and $E_i = \{R_I^{k}...', R_{i-1}^{k}\}$ is the set results of the tests run so far.

The scoring function $f$ can be implemented in several ways. For example, it can be realised as a single module capable of automatically putting the various inputs in relation, and generating scores for the whole set $T,$ e.g., a multi-modal neural network, outputting a posterior probability over the set of $T$ (or any derived ranking) given the inputs. A similar approach can be also designed to output independent scores, i.e., scoring each test independently given the inputs. Alternatively, the function can be also designed as a cooperation of single modules analysing the different inputs, with a single main module crossing such post-processed information to score tests accordingly.

The test scoring function $f$ can in some embodiments be implemented as a learning-to-rank approach, where a machine learning (ML) algorithm output ranking scores. By way of a nonlimiting illustrative example, the ML algorithm can comprise a deep neural network suitably structured to fuse all the input information and to provide a relevance score as output. *See, e.g.,* Pang et al., "DeepRank: A New Deep Architecture for Relevance Ranking in Information Retrieval", In Proceedings of the 2017 ACM on Conference on Information and Knowledge Management (November 2017 (CIKM '17)). In the network, questions and answers can be encoded as natural-language inputs, for example, with language models. Log files are suitably encoded by training a language model over the set of all existing log files.

To train the ML algorithm, training data can be generated by building a parallel corpus from recorded interactions between service engineers and customers, with data on the remote tests run during such interactions, and related log files of the systems. Each customer-SE dialog turn is paired with the immediately subsequent remote test that is run by the SE, by way of time signatures of the recorded interactions and test runs. A similar approach can be applied to align log files from the system. Training data can be obtained from historical data, with a minor overhead for input alignment; at the same time, the training data can be extended with new data coming from real-time interactions, where the alignment is resolved automatically at runtime.

Advantageously, this will provide the SE with a smaller set of remote tests to choose from, when diagnosing a medical device **120.** These tests will be, in most of the cases, strictly correlated with the treated malfunction, thanks to the contribution of user's answer, information in the LFOD and history of previous test results. The SE will spend less time running unrelated and useless test, and therefore the SE will be able to drill down the malfunction more quickly.

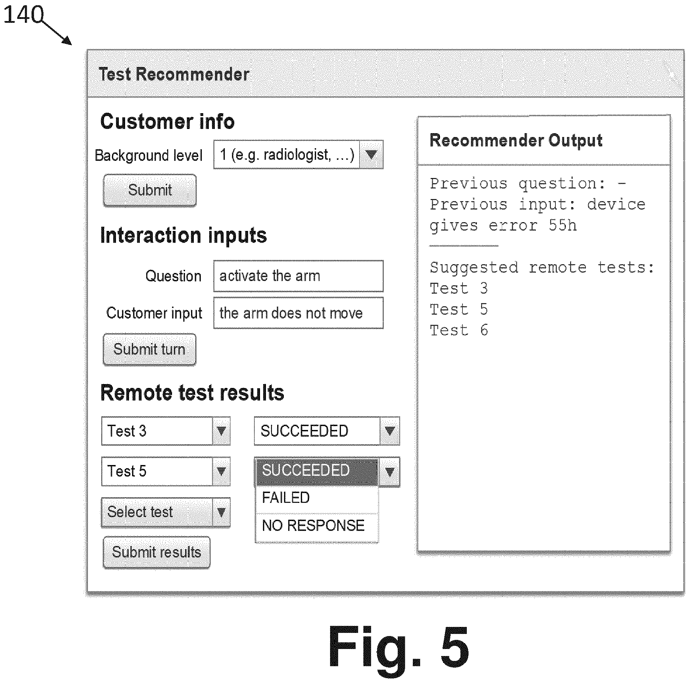

The test recommender module **302** can be extended to also suggest a question for the customer to the SE. The question should match the customer background profile b. This is used to select a collection of profiled question templates $Q_b$ from a set of templates $Q$ (i.e., the templates **134).** The background can be provided through the same test recommender module **302** interface I, in a dedicated field. Again, the field can be a free text slot, or a drop-down menu with fixed values. As a variant, backgrounds could be grouped in general level of expertise, e.g., LEVEL-0 for totally non experienced customers like secretaries, LEVEL-1 for medical personnel with some experience, LEVEL-3 for technical personnel, and so on. In this case, then, the number of levels would be equal to $|Q|$, and a background b would correspond to a level of expertise. Interaction with the customers can be quite complex, and the actual speaking customer may change over time. The categorisation in levels would help in such cases, making easier for the SE to identify a level of technical preparation of the customer. Fig. 5 shows an example of such an interface I (i.e., the UI **140).**

Given a background level *b,* profiled questions are then selected from the collection $Q_b$ with a *retrieval-based* strategy (see, e.g., Wang, H. a. (2013). A Dataset for Research on Short-Text Conversations. Proceedings of the 2013 Conference on Empirical Methods in Natural Language Processing (pp. 935--945). Seattle, Washington, USA: Association for Computational Linguistics). A score z is computed for each question candidate, and the one with the highest score is selected. The question selection process can then be formalised as a function g providing a distribution score over the set of possible questions, such as Equation 4:

$$g(a_i, L, R_i^k, A_i, E_i) = z_1, \dots, z_r, \qquad (4)$$

where $r = |Q_b|$, or alternatively, score each question

$q^p \in Q_b$, with $p = 1 \dots r,$ namely

$$g(a_i, L, R_i^k, A_i, E_i, q^p) = z_p.$$

Analogous to the implementation off the function g can be implemented in several ways, as a single algorithm matching all the inputs, or as a cooperation of different modules, with a single arbiter merging the information and computing the final score(s). Moreover, *f* and g may be implemented as a single function, producing two parallel outputs, namely the test scores and the question scores.

Advantageously, the SE is helped in formulating questions better understandable to the customer. This should help in getting the best of the information out of the customer about the current system behaviour, with a twofold effect. First, more accurate customer information will be provided to the test recommender module **302,** helping in suggesting more suitable remote tests. Secondly, the malfunction will be detected more quickly.

In some embodiments, the test recommender module **302** can be provided with an interface on customer side. This interface is used to increase the level of involvement and interaction, making the customer more aware of what is going on during the maintenance process. The numbers and types of accessible functionalities through the customer interface can vary depending on design choices, and/or on the customer background. For example, the customer interface may show the SE's suggested questions and may allow to prompt for replies for a user with a non-technical background. In addition to this, the customer interface may also allow to check the results of the remote tests, or even to suggest some of them to the SE, in the case of a more technical customer. Indeed, the SE would still behave as a man-in-the-middle, filtering the inputs and outputs of both customer and the test recommender module **302.**

Advantageously, the customer interface allows for a more agile interaction between the SE, the customer, and the medical device **120,** enabling for a higher parallelisation of the operations. The SE would be able to interact with the medical device **120** while asynchronously receiving direct input from the customer (i.e., not only from the phone). Second, a customer with a technical background would be able to observe the medical device **120** outputs, and thus integrate the SE's knowledge by suggesting possible tests and operations on a stronger basis.

In some embodiments, the test recommender module **302** can also be exposed directly to a customer with technical background, i.e., a BioMed Engineer, for a complete end-to-end troubleshooting and diagnosing process. The SE would be not present anymore, with the BioMed Engineer interacting directly with the Test Recommender through the interface I, suitably adjusted to meet a good level of interaction with a remote user. The questions $q_i$ and the answers $a_i$ would be provided in a sort of chat channel, where the answers may be free-text or multiple choices, depending on the state of the knowledge. The BioMed Engineer would be able to select a set of suggested remote tests to run, and to consult the results of such tests on the output display.

Advantageously, BioMed Engineers can diagnose a malfunctioning device on their own, without the need of a SE as a man-in-the-middle. This will have two further effects. First, the workload on the SEs would be reduced for many cases, making them more available for solving more complicated cases. Second, it will streamline the whole diagnostic process even in presence of a SE. In fact, a BioMed could potentially run some diagnostics before calling the SE, if needed. The diagnostic process on the SE side would then start from an advanced state, with more prior knowledge on the system status, shortening the intervention duration.

In other embodiments, the test recommender module **302** can be designed to be able to automatically select only of portion of all the available LFOD, to decrease the computational load of transferring the entire log files for a given system, as well as refining the information used for the recommendation process. The entire LFOD of a system is composed, in fact, by a lot of different log sources, e.g., logs of some sub-components, data dumps, images, and so on. Relying on the whole set of them may result in an excess of processing, which may also lead to poorer recommendation results.

A function can be defined as $L = \{ L^1, L^2, ..., L^N\}$, where each $L^n$ is a separate sub-set of the LFOD, e.g., $L^1$= main system logs, $L^2$= images logged while in operation, $L^3$= data dumps, and so on - we can extend the Test Recommender with a LFOD selection function $l$ that selects one of the log set in $L$, Equation (5) is generated:

$$l(a_i, R_i^k, A_i, E_i) = L_i^n \qquad (5)$$

The function $l$ is employed at every interaction turn $i$. automatically selecting one of the possible log sources given the progress of the diagnosis, i.e., represented by $a_i$, $R_i^j$, $A_i$ and $E_i$. The selected log $L_i^n$ would be then used in the functions $f$ or $g$ instead of $L$ for the turn $i$. The function $l$ may be designed also to return more than one possible subset $L^n$. In this perspective, the function may be also smartly built or trained to first access a minimal or generic set of LFOD, and then access more heavy or complex ones according to the necessity of the diagnosis.

Advantageously, the overall computational load can be reduced both for the LFOD transfer process, and for the analysis process. In fact, the amount of data to access or transfer, i.e., the size of the selected log files, will be reduced to only a portion of the whole LFOD. Moreover, the recommending function $f$ (or $g$) will have to analyse a smaller, albeit possibly more accurate and discriminant source of information, resulting in a lesser usage of computational power.

A non-transitory storage medium includes any medium for storing or transmitting information in a form readable by a machine (e.g., a computer). For instance, a machine-readable medium includes read only memory ("ROM"), solid state drive (SSD), flash memory, or other electronic storage medium; a hard disk drive, RAID array, or other magnetic disk storage media; an optical disk or other optical storage media; or so forth.

The methods illustrated throughout the specification, may be implemented as instructions stored on a non-transitory storage medium and read and executed by a computer or other electronic processor.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

## Claims

1. A non-transitory computer readable medium **(107, 111)** storing instructions executable by at least one electronic processor **(101, 113)** to perform a call intake method **(200),** the method comprising:

   receiving caller-provided information describing an issue related to a functioning of a medical device from the caller;
   retrieving device log data automatically generated by the medical device;
   determining scores for diagnostic tests **(132)** of a set of diagnostic tests for diagnosing the issue with the medical device wherein the scores are determined based on the retrieved device log data and the caller-provided information; and
   outputting, on a display device **(105)** of a service engineer (SE) electronic device **(102)** operable by the SE, a ranked list **(142)** of one or more recommended diagnostic tests of the set of diagnostic tests based on the scores.

2. The non-transitory computer readable medium **(107, 111)** of claim 1, wherein the receiving of the caller-provided information includes:

   providing a list of questions **(136)** on the SE electronic device **(102)** or a caller electronic device **(104)** operable by the caller; and
   receiving, at the SE electronic device, inputs indicative of answers to the provided questions from the caller.

**3.** The non-transitory computer readable medium **(107, 111)** of claim 2, wherein the method **(200)** includes:

   ranking the list of questions **(136)**; and
   presenting the ranked list of questions on the SE electronic device **(102)** or the caller electronic device **(104).**

**4.** The non-transitory computer readable medium **(107, 111)** of claim 3, wherein ranking the list of questions **(136)** includes:

   inputting the retrieved device log data into question templates **(134)** stored in a database **(111)**; and
   ranking the list of questions based on the input of the retrieved data into the question templates.

**5.** The non-transitory computer readable medium **(107, 111)** of either one of claims 3 and 4, wherein ranking the list of questions **(136)** includes:

   receiving information regarding a level of expertise of the caller; and
   filtering out questions from the list of questions based on the level of expertise of the caller.

**6.** The non-transitory computer readable medium **(107, 111)** of any one of claims 1-5, wherein the instructions further include:

   receiving results for one or more performed diagnostic tests of the one or more recommended diagnostic tests;
   removing the performed diagnostic tests from the set of diagnostic tests to generate a set of remaining diagnostic tests;
   updating scores **(138)** for the diagnostic tests **(132)** of a set of remaining diagnostic tests, the updated scores being determined based on the retrieved device log data and the caller-provided information and further based on the results for one or more performed diagnostic tests; and
   outputting, on the display device **(105)** of the SE electronic device **(102),** an updated ranked list (142) of one or more recommended diagnostic tests of the set of remaining diagnostic tests based on the updated scores.

**7.** The non-transitory computer readable medium **(107, 111)** of any one of claims 1-6, wherein the instructions further include:
   remotely controlling the medical device **(120)** to perform the one or more performed diagnostic tests whereby the results are received from the medical device.

**8.** The non-transitory computer readable medium **(107,** **111)** of any one of claims 1-7, wherein the instructions further include:

   adding a required experience level to each test in the ranked list **(142)** of tests; and
   transmitting the ranked list of tests to a plurality of SEs having an experience level to perform the tests in the ranked list of tests.

**9.** A system, comprising:

   a medical device **(120)**; and
   at least one electronic processor **(101, 113)** programmed to:

      receive caller-provided information describing an issue related to a functioning of a medical device from the caller;
      retrieve device log data automatically generated by the medical device;
      determine scores for diagnostic tests **(132)** of a set of diagnostic tests for diagnosing the issue with the medical device wherein the scores are determined based on the retrieved device log data and the caller-provided information; and
      output, on a display device **(105)** of a service engineer (SE) electronic device **(102)** operable by the SE, a ranked list **(142)** of one or more recommended diagnostic tests of the set of diagnostic tests based on the scores.

**10.** The system of claim 9, wherein the at least one electronic processor **(101, 113)** is programmed to receive the caller-provided information by:

   providing a list of questions **(136)** on the SE electronic device **(102)** or a caller electronic device **(104)** operable by the caller; and
   receiving, at the SE electronic device, inputs indicative of answers to the provided questions from the caller.

**11.** The system of claim 10, wherein the at least one electronic processor **(101,113)** is further programmed to:

   rank the list of questions **(136)**; and
   present the ranked list of questions on the SE electronic device **(102)** or the caller electronic device **(104).**

**12.** The system of claim 11, wherein the at least one electronic processor **(101, 113)** is programmed to rank the list of questions **(136)** by:

   inputting the retrieved device log data into question templates **(134)** stored in a database **(111)**;

and
ranking the list of questions based on the input of the retrieved data into the question templates.

13. The system of either one of claims 11 and 12, wherein the at least one electronic processor **(101, 113)** is programmed to rank the list of questions **(136)** by:

receiving, from the caller, an indication of a level of expertise of the caller; and
filtering out questions from the list of questions based on the level of expertise of the caller.

14. The system of any one of claims 9-13, wherein the at least one electronic processor (101, 113) is further programmed to:

receive results for one or more performed diagnostic tests of the one or more recommended diagnostic tests;
remove the performed diagnostic tests from the set of diagnostic tests to generate a set of remaining diagnostic tests;
update scores **(138)** for the diagnostic tests **(132)** of a set of remaining diagnostic tests, the updated scores being determined based on the retrieved device log data and the caller-provided information and further based on the results for one or more performed diagnostic tests; and
output, on the display device **(105)** of the SE electronic device **(102),** an updated ranked list **(142)** of one or more recommended diagnostic tests of the set of remaining diagnostic tests based on the updated scores.

15. The system of any one of claims 9-14, wherein the at least one electronic processor **(101, 113)** is further programmed to:
remotely control the medical device (120) to perform the one or more performed diagnostic tests whereby the results are received from the medical device.

Fig. 1

Fig. 2

**Fig. 3**

EP 4 191 605 A1

**140**

**Test Recommender**

**Interaction inputs**

Question | activate the arm

Customer input | the arm does not move

[ Submit turn ]

**Remote test results**

Test 3 | ▼ | SUCCEEDED | ▼

Test 5 | ▼ | SUCCEEDED | ▼
                 FAILED
Select test | ▼ | NO RESPONSE

[ Submit results ]

**Recommender Output**

```
Previous question: -
Previous input: device
gives error 55h

Suggested remote tests:
Test 3
Test 5
Test 6
```

A

---

**Test Recommender**

**Interaction inputs**

Symptom

Part | Behaviour

Arm | ▼ | Stuck | ▼
                 Do not reset
[ Submit turn ] | Emit noise

**Remote test results**

Test 3 | ▼ | SUCCEEDED | ▼

Test 5 | ▼ | SUCCEEDED | ▼
                 FAILED
Select test | ▼ | NO RESPONSE

[ Submit results ]

**Recommender Output**

```
Previous question: -
Previous input: error 55h

Suggested remote tests:
Test 3
Test 5
Test 6
```

B

# Fig. 4

Fig. 5

Europäisches
Patentamt

European
Patent Office

Office européen
des brevets

# EUROPEAN SEARCH REPORT

**Application Number**

EP 22 16 5163

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2002/042717 A1 (HAHN GUENTER [CN]) 11 April 2002 (2002-04-11) * The whole document, in particular: Paragraphs [0005], [0006], [0014], [0032] – [0048]; Figures 3 – 7. * ----- | 1–15 | INV. G16H40/40 G16H40/67 |
| A | US 2006/085689 A1 (BJORSNE TORD [DE]) 20 April 2006 (2006-04-20) * The whole document, in particular: Paragraphs [0001], [0006], [0012] – [0020], [0031] – [0046]. * ----- | 1–15 | |

**TECHNICAL FIELDS SEARCHED (IPC)**

G16H

The present search report has been drawn up for all claims

| Place of search | Date of completion of the search | Examiner |
|---|---|---|
| Munich | 9 September 2022 | Nagele, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 16 5163

09-09-2022

| Patent document cited in search report | | | Publication date | Patent family member(s) | | | Publication date |
|---|---|---|---|---|---|---|---|
| US 2002042717 | A1 | | 11-04-2002 | CN | 1359090 | A | 17-07-2002 |
| | | | | DE | 10047547 | A1 | 25-04-2002 |
| | | | | JP | 2002157362 | A | 31-05-2002 |
| | | | | US | 2002042717 | A1 | 11-04-2002 |
| US 2006085689 | A1 | | 20-04-2006 | CN | 1763720 | A | 26-04-2006 |
| | | | | DE | 102005046388 | A1 | 13-04-2006 |
| | | | | NL | 1030059 | C2 | 24-10-2006 |
| | | | | US | 2006085689 | A1 | 20-04-2006 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PANG et al.** DeepRank: A New Deep Architecture for Relevance Ranking in Information Retrieval. *In Proceedings of the 2017 ACM on Conference on Information and Knowledge Management,* November 2017

- **WANG, H.** A Dataset for Research on Short-Text Conversations. *Proceedings of the 2013 Conference on Empirical Methods in Natural Language Processing,* 2013, 935-945